# EUROPEAN PATENT APPLICATION

(11) **EP 1 978 106 A1**
(43) Date of publication of application: **08.10.2008**
(21) Application number: 07105836.6
(22) Date of filing: 07.04.2007
(51) Int. Cl.: C12Q 1/68

(54) **Detection of ESR1 amplification in endometrium cancer and ovary cancer**

(71) Applicant: Universitätsklinikum Hamburg-Eppendorf, 20246 Hamburg (DE)
(72) Inventor: Simon, Ronald, 24568 Kaltenkirchen (DE); Sauter, Guido, 22397 Hamburg (DE); Terracciano, Luigi, 4059 Basel (CH); Holst, Frederik, 22299 Hamburg (DE); Lebeau, Annette, 23923 Herrnburg (DE); Turzynski, Andreas, 23923 Herrnburg (DE)
(74) Representative: UEXKÜLL & STOLBERG

(57) **Abstract**

The present invention relates to an in-vitro method of identifying a tumor resulting from a proliferative disease of the endometrium or ovary as responsive to anti-estrogen treatment. Further, the invention relates to an in-vitro method of identifying a candidate patient with a proliferative disease of the endometrium or ovary as suitable for anti-estrogen treatment. In a further aspect, the invention provides an in-vitro method of identifying an individual with a non-cancerous proliferative disease of the endometrium or ovary who is at risk of developing endometrial or ovarian cancer. The invention also provides kits for performing the above methods.

## Description

### FIELD OF THE INVENTION

The present invention relates to an in-vitro method of identifying a tumor resulting from a proliferative disease of the endometrium or ovary as responsive to anti-estrogen treatment. Further, the invention relates to an in-vitro method of identifying a candidate patient with a proliferative disease of the endometrium or ovary as suitable for anti-estrogen treatment. In a further aspect, the invention provides an in-vitro method of identifying an individual with a non-cancerous proliferative disease of the endometrium or ovary who is at risk of developing endometrial or ovarian cancer. The invention also provides kits for performing the above methods.

### BACKGROUND OF THE INVENTION

Endometrial cancer is the most common gynecologic malignancy in developed countries. In the EU, it accounts for about 6% of cancers in women, with an overall incidence 17/100000/year and a death rate of 3,5/100000/year 1. Estrogen-dependent endometrial tumors (type I) make up the vast majority (>90%) of endometrial tumors. They are typically low grade, i.e. well or moderately differentiated and predominantly of the endometroid type. Patients with this form of cancer frequently are obese, diabetic, nulliparous, and hypertensive or have late menopause. Type I tumors are frequently associated with endometrial hyperplasia, in particular atypical hyperplasia. Unopposed estrogenic stimulation, and overexpression of estrogen receptor (ER), is generally seen as the driving force behind this group of tumors (Silverberg, S.G. et al. Tumours of the uterine corpus: Epithelial tumors and related lesions. in WHO classification of tumours (eds. Tavassoli, F.A. & Devilee, P.) 221-249 (IARC Press, Lyon, 2003). However, unlike in breast cancer, the results of anti-estrogenic therapy in early and advanced stages of disease have been predominantly disappointing (Thigpen, T., et al. (2001), J Clin Oncol, 19, 364-7). In particular the success of Tamoxifen, a potent ER inhibitor that is widely prescribed for treatment of ER positive breast cancer, appears to be limited in endometrial cancer. Evidently, it would be favorable to provide means to identify patients which respond to agents such as Tamoxifen before starting a treatment regime.

Ovarian cancer is the fifth leading cause of cancer death in women, and the second most commonly diagnosed gynecologic malignancy. The economically advanced countries show the highest rates. The cause of non-familial ovarian cancer is usually unknown. However, recent studies have shown an increased risk in postmenopausal women treated with high-dose estrogen replacement therapy for 10 years or later. The protective effects of pregnancies and of oral contraception suggest a direct role for ovulation in causing the disease, but no convincing mechanism linking the risk factors with malignant transformation has been proposed (Lee, K.R., et al (2003) in WHO classification of tumors (eds. Tavassoli, F.A. & Devilee, P.) 221-249 (IARC Press, Lyon, 2003).

It has now been surprisingly found that amplification of the ESR1 gene located at 6q25.1 and encoding the alpha isoform of the estrogen receptor is a common feature of both cancerous and non-cancerous proliferative diseases of the endometrium and the ovary, such as endometrial or ovarian cancer. ESR1 gene amplification was found in 22.7% of endometrial cancers and 7,5% of ovarian cancers examined in this respect, and it occurred in all histological subtypes. ESR1-amplified tumors of the endometrium and the ovary show a particular strong response to Tamoxifen and similar agents frequently used in anti-estrogen therapy. As a consequence, detection of ESR1 amplification is of significant clinical relevance and may be used in diagnosis and estimation of prognosis and also as a tool for making decisions as to the specific treatment protocol to be used with a particular patient suffering from a proliferative disease of the endometrium or ovary, such as endometrial or ovarian cancer.

Amplification of genomic DNA is the result of a selection process aiming at facilitating tumor cell growth, e.g. by high level overexpression of genes that otherwise would be growth rate limiting. Amplified genes, therefore, are likely to be vitally important for tumor cells and represent particular attractive targets for new gene specific therapies. For example, in breast cancer, more than 30 regions of amplification have been detected by means of classical comparative genomic hybridization (CGH), see for example, O'Connell, et al. (2003), Breast Cancer Res Treat, 78: 347-357.

### DESCRIPTION OF THE FIGURES

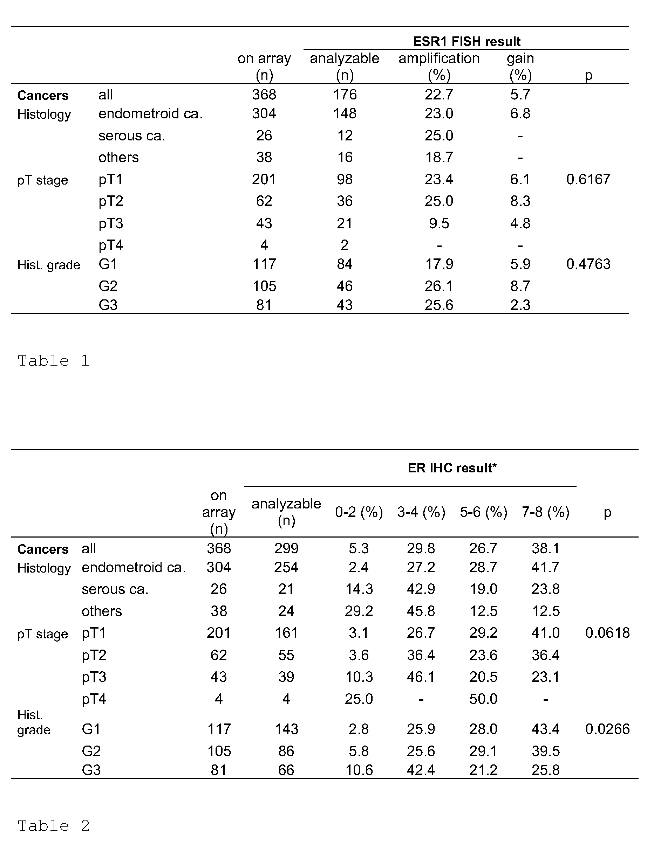

Figure 1 shows in table 1 the results from FISH analysis of ESR1 amplification in endometrial cancer. In table 2, the results from immunohistochemistry analysis of estrogen receptor expression in endometrial cancer are shown.

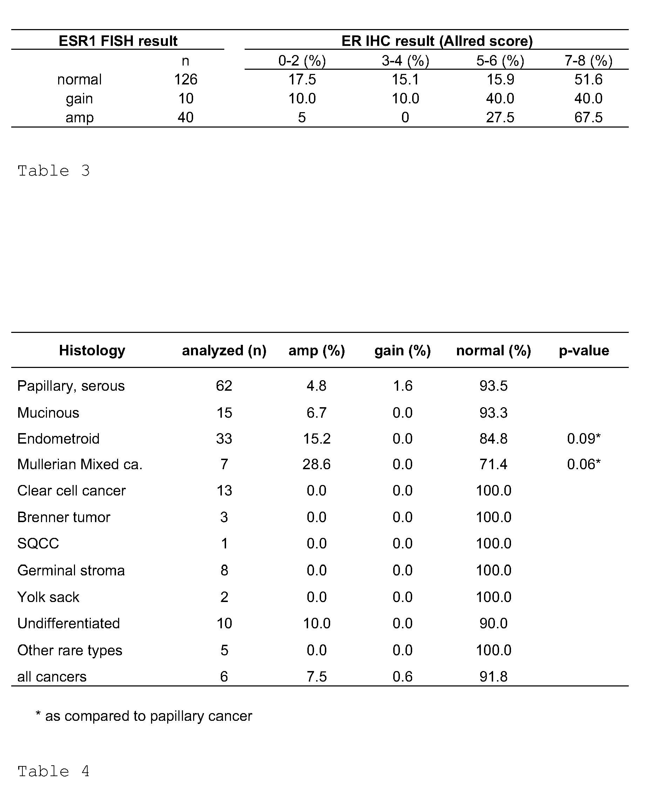

Figure 2 shows in table 3 the association between ESR1 amplification and estrogen receptor expression in endometrial cancer. In table 4, the results from FISH analysis of ESR1 amplification in ovarian cancer are depicted.

### SUMMARY OF THE INVENTION

Estrogens belong to the group of steroid hormones. The three major naturally occurring estrogens in women are estradiol, estriol and estrone. From puberty to menopause, estrogen production mainly takes place in the ovaries. After menopause, when the ovaries no longer produce estrogens, body fat is the primary source for these hormones. Like other steroid hormones, estrogens act as signalling molecules and exhibit their function by binding to estrogen receptors which are present inside cells of those tissues which are targets for estrogen regulation. Two different human estrogen receptors occur which are designated estrogen receptor alpha isoform (or ER-alpha) and estrogen receptor beta isoform (ER-beta). The isoforms are encoded by different genes, ESR1 and ESR2, respectively, which are found at different chromosomal locations, and numerous mRNA splice variants exist for both receptors in both diseased and normal tissue (see for example, Deroo & Korach (2006), Journal of Clinical Investigation 116: 561-570).

Like all steroid receptors, the estrogen receptors (i.e. the alpha and beta isoform) exhibit a modular structure, with discrete regions of the protein (domains) responsible for transcriptional activation, DNA binding, nuclear localization, ligand binding, and dimerization (see Peters and Khan (2003), Mol Endocrin 13(2):286-296). ER-alpha and ER-beta share a high degree of homology in the ligand binding (AF-2) and DNA-binding domains, but differ in the activation function (AF-1) domain. Comparison of the AF-1 domains suggests that the activity on estrogen response elements is much stronger in ER-alpha as compared to ER-beta (Cowley, S.M., et al. (1999), J Steroid Biochem Mol Biol 69: 165-175). Although comparatively little is known about the function and clinical significance of ER-beta, it is generally believed that ER-beta counteracts the function of ER-alpha and leads to a reduction of estrogen-stimulated proliferation (Omoto, Y., et al. (2003), Oncogene 22: 5011-5020).

The human estrogen receptor alpha is encoded by the ESR1 gene which maps to 6q25.1 of human chromosome 6. The nucleotide sequence of SEQ ID NO:1 shows the ESR1 coding sequence together with approximately 1 Mb of sequence located upstream (at the 5'-end of the sequence) of the ESR1 coding sequence and approximately 1,38 Mb of sequence located downstream (at the 3'-end of the sequence) of the ESR1 coding sequence. The nucleotide sequence of the ESR1 gene starts with the nucleotide in position 1048135 of the sequence shown in SEQ ID NO:1 and ends in position 1343855 of the sequence shown in SEQ ID NO:1. As used herein, the terms "ESR1 gene" and "ESR1 coding sequence" are used interchangeably and refer to the genomic sequence transcribed and spliced to the mRNA depicted in SEQ ID NO:2 without promoter and enhancer structures which might be associated to this DNA entity. The ESR1 gene sequence is also available under NCBI GenBank ID NT_025741.14 where the assembled nucleotide sequence of human chromosome 6 is provided. In the sequence portion designated "Human Genome Build 36", the ESR1 gene covers nucleotides 152170379 to 152466099. The ESR1 gene comprises several introns which are spliced out after transcription. The nucleotide sequence of the spliced ESR1 mRNA sequence is depicted in SEQ ID NO:2 and is available under NCBI GenBank ID NM_000125. The corresponding amino acid sequence of the estrogen receptor alpha protein is depicted in SEQ ID NO:3 and is also available under NCBI GenBank ID NM_000125. There are numerous allelic variants of the ESR1 gene, see for example, Modugno et al. 2001, Clin Cancer Res. 7(10):309 or Mansur Ade et al. 2005, Arch Med Res. 36(5):511.

The estrogen receptors (alpha or beta isoform) are normally located in the nucleus of the target cell. According to the accepted model of steroid hormone action, the estrogen receptors are in an inactive state in the absence of hormone. When estrogen passes into the nucleus, the estrogen receptors bind to estrogen. Upon estrogen binding, the receptors form dimers which then bind to estrogen response element DNA sequences directly or indirectly through protein-protein interactions with activator protein 1 (AP1) or SP1 sites in the promoter region of estrogen-responsive genes. This binding results in the recruitment of co-regulatory proteins (co-activators or co-repressors) to the promoter, thereby leading to an increased or decreased gene expression. The altered gene expression can influence cell behavior in different ways, depending on the tissue type involved. In some target tissues, such as breast tissue, the main effect of estrogen in healthy women is to induce cell proliferation. For instance, estrogen causes the proliferation of cells lining the milk glands in order to prepare milk production. Aside from homodimeric receptors consisting of two alpha units or two beta units, mixed dimers may also occur. Different tissues express the two isoforms in different proportions, and therefore have different responses to stimulation by estrogens.

Although the ability to promote proliferation of certain cell types lies within the normal functions of the estrogen molecule, it is also associated with an increased risk of developing cancer, such as breast cancer (Lawson J.S., et al. (1999), Lancet 354:1787-1788). Once cancerous cells have formed, it is highly undesirable to have signal molecules that trigger proliferation of these cells. As a matter of consequence, immunohistochemical detection of expression of the alpha isoform of the estrogen receptor is routinely performed in order to determine whether breast cancer cells of a given patient express estrogen receptor or not (Andersen, J. and Poulsen, H. S. (1989), Cancer, 64: 1901-1908). With breast cancer, it is known that more than two thirds of breast cancers show expression of the alpha isoform of the estrogen receptor at the time of diagnosis (Stierer, M., et al. (1993), Ann Surg, 218: 13-21). These cancers are generally referred to as estrogen-receptor-positive or ER-positive. In the remaining breast cancer cases, no estrogen-receptor alpha protein can be detected in the cells. These cancers are estrogen-receptor-negative or ER-negative. ER-positive breast cancers are currently treated by anti-estrogen therapy with so-called selective estrogen receptor modulators (SERMs) and/or aromatase inhibitors. For example, an estrogen antagonist like Tamoxifen is frequently applied to the patient with ER-positive breast cancer, normally after surgical removal of the tumor.

In pending European Patent application 06116106.3, it has been shown that it is possible to identify a subgroup within the group of ER-positive patients suffering from breast cancer, which show a particular good response to anti-estrogen therapy. It could be demonstrated that tumors of breast cancer patients that show amplification of the ESR1 gene on the genomic level exhibit a clearly enhanced response to anti-estrogen therapy with Tamoxifen. These patients were classified as particular suitable for anti-estrogen therapy, for example with Tamoxifen.

In the course of the present invention, it was now furthermore found that similar subgroups of tumors with amplified ESR1 gene can be identified with endometrial and ovarian cancer. Thus, the methods of the present invention allow prediction of the responsiveness of a tumor resulting from a proliferative disease of the endometrium or ovary to anti-estrogen treatment based on the evidence that the ESR1 gene is amplified. Such information can be useful in order to design an appropriate therapy schedule which is more tailored to the nature and molecular characteristics of the tumor. For example, in cases where a tumor shows a particularly good responsiveness to anti-estrogen treatment, for example, administration of Tamoxifen, the patient can be subjected to an anti-estrogen monotherapy, without the need to apply a concurrent chemotherapy. Alternatively, where chemotherapy is still part of the therapy, it might be possible to reduce the dosis of the chemotherapeutic agents while maintaining the same treatment results.

Amplification of the ESR1 gene is regularly associated with co-amplification of genomic sequence portions that frame the ESR1 gene on the human chromosome. It has been shown that it is possible to spot ESR1 gene amplification events both directly by detecting amplification of the ESR1 sequence and indirectly by detecting amplification of a nucleotide sequence which comprises or flanks the ESR1 gene. Thus, according to a first aspect of the invention, an in-vitro method of identifying a tumor which results from a proliferative disease of the endometrium or ovary as responsive to anti-estrogen treatment is provided. The method comprises the steps of
a) selecting a nucleotide sequence portion of the nucleotide sequence of SEQ ID NO:1;
b) detecting in a cell sample from said tumor whether said nucleotide sequence portion is amplified in the genome of said tumor cells;
c) classifying said tumor as responsive to anti-estrogen treatment, if the nucleotide sequence portion is amplified in the genome of said tumor cells.

According to a second aspect of the invention, an in-vitro method of identifying a candidate patient with a tumor resulting from a proliferative disease of the endometrium or ovary as suitable for anti-estrogen treatment is provided. The method comprises the steps of
a) selecting a nucleotide sequence portion of the nucleotide sequence of SEQ ID NO:1;
b) detecting in a cell sample from said tumor whether said nucleotide sequence portion is amplified in the genome of said tumor cells;
c) classifying said patient as one that is suitable for anti-estrogen treatment, if the nucleotide sequence portion is amplified in the genome of said tumor cells.

According to a preferred embodiment of the invention the tumor results from endometrial cancer or ovarian cancer. However, ESR1 amplification can not only be detected in endometrium or ovary cancer, but also in a significant part of benign proliferative diseases of the endometrium and ovary, such as complex endometrial hyperplasias with atypia.

Gene amplifications - like mutations - constitute genetic alterations. Generally, amplifications modulate gene activity by massively overexpressing mRNA and resulting protein. In normal epithelial tissue, the function of the alpha isoform of the estrogen receptor resides in the reception and transduction of a signal facilitating cell proliferation. ESR1 amplification has a similar impact on cell proliferation in non-cancerous and cancerous proliferative diseases. Increased proliferation rates increase the risk to acquire additional genetic alterations and, in turn, the risk of developing cancer. Thus, ESR1 amplification can indicate a non-cancerous proliferative disease of the endometrium or ovary with an increased potential for malignant transformation. ESR1 amplification status analysis may serve as a prognostic marker in patients with such non-cancerous proliferative diseases of the endometrium or ovary described herein.

According to a further aspect, the invention therefore provides an in-vitro method of identifying an individual with a tumor resulting from a non-cancerous proliferative disease of the endometrium or ovary who is at risk of developing endometrial or ovarian cancer, respectively. The methods comprises the steps of
a) selecting a nucleotide sequence portion of the nucleotide sequence of SEQ ID NO:1;
b) detecting in a cell sample from said tumor whether said nucleotide sequence portion is amplified in the genome of said tumor cells;
c) classifying said individual as one that is at risk of developing endometrial or ovarian cancer, respectively, if the nucleotide sequence portion is amplified in the genome of said tumor cells.

The methods according to the invention are in vitro methods which utilize samples comprising cells and/or tissue from the endometrium or ovary. The cells and tissues are derived from a region of concern, in particular from a tumor. The source of the tissue sample may be, for example, solid tissue from a fresh, frozen and/or preserved tissue sample, biopsy, cytological smear, or aspirate. Preferably, the samples are derived from an endometrium or ovary biopsy. An endometrium or ovary biopsy involves removing cells or tissue for further molecular and/or histological examination. For example, endometrium or ovary biopsy samples are normally used to determine whether cancerous cells are present in the endometrium or ovary of a patient. Hitherto, biopsy and subsequent pathological analysis is the only definitive way to confirm endometrium or ovary cancer. Nevertheless, it is anticipated that detection of ESR1 amplification is also possible in blood samples. For this purpose, tumor cells are isolated from peripheral blood and subjected to amplification detection. Methods for tumor cell isolation from blood include filtration procedures ("Isolation by size of epithelial tumor cells (ISET)", described in Vona et al. (2000) Am J Pathol 156:57) or immuno-magnetic (Brandt et al. (1998), Int J Cancer 76:824) or flow cytometric approaches (Wong et al. (1995), Br J Surg 82:1333). Furthermore, detection of ESR1 amplification should also be possible in cells obtained from bone marrow cells.

In the prior art, different methods for obtaining an endometrium or ovary biopsy or cytologic smear are known. These biopsy methods are well known in the art and have been described in numerous publications and standard textbooks (see for example, S.K. Rosevear, Handbook of gynaecological management, Blackwell Science, 2002 or M. James et al., Obstetrics and Gynaecology: A Problem-Solving Approach, Bailliere Tindall, 1999). For example, an endometrium biopsy may be obtained through the use of a Pipelle or Norak curette. The curette is inserted through the cervix into the uterine cavity and uterine tissue is removed. Endometrium smears may be obtained by cytobrush or uterine lavage techniques. Ovarian biopsies may be obtained by laparoscopic biopsy techniques.

The samples to be examined are obtained from a patient suffering from a proliferative disease of the endometrium or ovary. A proliferative disease of the endometrium or ovary refers to any state of the endometrial or ovarian tissue, respectively, which is associated with an abnormal and/or uncontrolled cell proliferation. In such proliferative disease, a tumor is formed in the endometrium or ovary, i.e., an abnormal mass of cells, that results from excessive cell division and performs no useful body function. Tumors of the endometrium or ovary are distinguished in benign tumors and malignant tumors. Accordingly, the proliferative endometrial or ovarian disease which leads to tumor formation may be a benign or a malignant disease.

Malignant tumors are generally cancerous which means that the patient is afflicted with endometrium or ovary cancer. Cancer has the potential to invade and destroy neighboring tissues and create metastases. The main types of endometrium cancer are endometroid, serous and clear cell adenocarcinoma. The main types of ovary cancer are serous, mucinous, endometroid and clear adenocarcinoma. The diagnosis and classification of endometrium or ovary cancers is well known in the art and discussed in numerous publications (see, for example, Tavassoli, F.A., et al. (2003), World Health Organization: Tumours of the Breast and Female Genital Organs, WHO/IARC Classification of Tumours).

Benign tumors are characterized in that they do not invade neighboring tissues and do not spread metastases. Normally, benign tumors do not recur after surgical removal of the tumor tissue. As used herein, benign tumors result from non-cancerous proliferative endometrial and ovarian diseases. Such non-invasive diseases also comprise pre-cancerous conditions which are known to frequently result in a cancerous disease at a later stage. According to a further aspect of the invention, the proliferative endometrial or ovarian disease is selected from the group of benign diseases consisting of endometrial hyperplasias (simple hyperplasia without atypia, complex hyperplasia without atypia, simple atypical hyperplasia, complex atypical hyperplasia) and ovarian tumors of low malignant potential (serous, mucinous, endometroid, and clear cell borderline tumors as well as Brenner borderline tumor). These conditions are known in the field of medicine and are moreover described in detail in standard textbook and numerous publications (see, for example, Tavassoli, F.A., et al. (2003), World Health Organization: Tumours of the Breast and Female Genital Organs, WHO/IARC Classification of Tumours).

According to the invention, it was found that individuals with a proliferative disease associated with a tumor having an amplified ESR1 gene in the genome of the tumor cells are particularly suitable for anti-estrogen therapy, for example, by administration of Tamoxifen. The tumors of these patients have shown to be particularly responsive to anti-estrogen therapy. For the purpose of the present invention, "responsive" in the context with a tumor or a patient means that a beneficial clinical reaction to the particular applied treatment is obtained, which leads to an improvement of the disease state with respect to said tumor or said patient. Likewise, particularly responsive means that the beneficial clinical reaction is stronger when compared to tumors of patients without ESR1 amplification (for example, ER-positive lacking amplification of the ESR1 gene or ER-negative) being subjected to the same treatment.

In relation to a tumor, the beneficial clinical reaction may comprise reduction of the tumor size, stabilization of the tumor size by slowing of growth and/or reduction of the tendency to spread metastases (in case of a malignant tumor). Preferably, a tumor which is responsive to anti-estrogen treatment will be reduced in size during or subsequent to therapy. In relation to a particular patient with a non-cancerous proliferative disease of the endometrium or ovary, the clinical reaction may also comprise the reduction of the risk of developing endometrial or ovarian cancer. In relation to a particular patient with endometrial or ovarian cancer the clinical reaction may also comprise delay or slowing of the disease progression, and in particular prolonging survival of the patient as compared to survival if not receiving any treatment.

An "anti-estrogen treatment" or "anti-estrogen therapy" means any measure that targets to interfere with the naturally occurring interaction between estrogen and the estrogen receptor, preferably the alpha isoform of the estrogen receptor. Specifically, anti-estrogen treatment or anti-estrogen therapy comprise measures which result in blocking the signal-transducing function of the estrogen receptor, which effects the estrogen-induced reactions, such as cell proliferation. Such measures comprise, for example, the administration of active agents or drugs that act by competitive inhibition of estrogen binding to an estrogen receptor, preferably the alpha isoform of the receptor. When administered in an therapeutically effective amount, these agents or drugs bind to the estrogen receptor, preferably the alpha isoform of the receptor, thereby blocking estrogen from binding to this receptor. According to the present invention, these compounds are referred to as "estrogen antagonists" (see below). Aside from estrogen antagonists, other current anti-estrogen strategies include destabilization and degradation of an estrogen receptor, preferably the alpha isoform, by administering a therapeutically effective amount of a selective estrogen receptor downregulator (e.g. Fulvestrant) or disruption of estrogen synthesis by administering a therapeutically effective amount of an aromatase inhibitor (e.g. Anastozole, Exemestan).

The term "therapeutically effective amount" refers to an amount of a drug effective to treat a disease or disorder in a mammal, preferably a human. In the case of a cancerous or non-cancerous proliferative disease of the endometrium or ovary, a therapeutically effective amount of a drug normally inhibits (i.e. slows to some extent and preferably stops) tumor growth and/or reduces tumor size. In the case of endometrial or ovarian cancer, it may also inhibit (i.e. slow to some extent and preferably stop) cancer cell infiltration into peripheral organs and inhibit (i.e. slow to some extent and preferably stop) tumor metastases. Furthermore, it may kill existing cancer cells. A therapeutically effective amount of a drug may also relieve one or more of the symptoms associated with a proliferative disease of the endometrium or ovary, such as endometrial or ovarian cancer. For therapy, efficacy of the drug administration can be measured, for example, by assessing the time to disease progression (TTP) and/or determining the response rate (RR). Methods for determining the efficacy of therapy are dependent on the particular disorder and moreover well-known to the person skilled in the art (Kelloff G.F. et al. (2005) Eur J Cancer 41: 491-501). The optimum dosis and treatment regimen for the particular agent administered as an anti-estrogen therapeutic are described in detail in the state of the art for several anti-estrogen drugs. For example, with respect to Tamoxifen, a therapeutically effective dosis may be between 10-100 mg/per day for a period of one to several years for example 2-5 years. Doses of about 60 mg/per day for a period of 5 years are reported in the literature (Kung et al. (2003), J Clin Endocrinol Metab, 88 (7) :3130) .

According to a preferred embodiment of the invention, the anti-estrogen treatment mentioned in the above methods comprises administration of an estrogen antagonist. According to the present invention, the term "estrogen antagonist" refers to a compound that binds to an estrogen receptor, and preferably to the alpha isoform of the estrogen receptor, (either in homodimeric or heterodimeric form), thereby inhibiting or substantially reducing the effect of the respective agonist (estrogen). The estrogen antagonist can be a competitive or non-competitive antagonist. A competitive estrogen antagonist competes with estrogen (or other agonists) for an estrogen receptor. By binding of the competitive estrogen antagonist to an estrogen receptor, the agonist estrogen is blocked from binding to the receptor. An example for such a competitive estrogen antagonist is Tamoxifen. In comparison, non-competitive antagonists antagonize the estrogen receptors by other means. For example, trilostane (Modrenal, Bioenvision) binds to the AF-1 domain of ER-alpha and ER-beta receptors in a non-competitive manner which is presumed to be allosteric. The AF-1 domain is involved in protein-protein interactions (but not estrogen binding) and trilostane binding thus contributes to modulation of receptor dimerization which is a prerequisite for activation (Puddlefoot J.R. et al. (2002), Int J Cancer 101: 17-22). Competitive or non-competitive estrogen antagonists can be found by common estrogen receptor binding assays, such as those described in the National Institutes of Health (NIH) publication no. 03-4504 (2002) including the protocols provided in the appendix.

According to a particularly preferred embodiment of the invention, the estrogen antagonist is selected from the group consisting of Tamoxifen (purchasable e.g. as Novaldex from Astra Zeneca, or from other manufactures, for example under the trade names Jenoxifen, Kessar, Nourytam, Tamobeta, Tamofen, Tamokadin, Tamoxasta, Tamox-GRY, Tamoxifen AL, Tamoxifen-biosyn, Tamoxifen cell pharm, Tamoxifen Heumann, Tamoxifen Hexal, Tamoxifen medac, Tamoxifen-ratiopharm, Tamoxigenat, Tamoximerck, Tamoxistad, Zemide, and the like), Raloxifene (purchasable e.g. as Revista from Eli Lilly), Clomifene (purchasable e.g. as Clomhexal from Hexal), Toremifene (purchasable as Fareston from GTx Inc.), Trilostane (purchasable as Modrenal from Bioenvision, UK only) or functional derivatives thereof. According to a particularly preferred embodiment of the invention, the estrogen antagonist is Tamoxifen or a functional derivative thereof. According to a further preferred embodiment, Tamoxifen is to be administered in combination with a hormone such as progesterone for treating proliferative endometrial diseases such as endometrial cancer in order to maximize therapeutic efficacy. Functional derivates are generally obtained from the above compounds by chemical modification. In the case of Tamoxifen, such derivates comprise for example 4-hydroxy-tamoxifen and 4-hydroxy-N-desmethyl-tamoxifen (Endoxifen).

Alternatively, the anti-estrogen treatment may comprise the administration of an agent which interferes with estrogen synthesis. By inhibiting, blocking or reducing the production of estrogens, a decrease in binding of estrogens (such as estradiol) to the estrogen receptors, preferably the estrogen receptor alpha, can be achieved. Agents which interfere with estrogen synthesis comprise, for example, aromatase inhibitors. Aromatases belong to the group of enzymes which comprise cytochrome P450 and catalyze the aromatization of androgens to estrogens, a key step in the production of estrogens. The inhibition of the aromatase enzyme results in reduced estrogen levels (hypoestrogenism). Aromatase inhibitors comprise compounds such as Anastrozole (purchasable as Arimidex from Astra Zeneca), Letrozole (purchasable as Femara from Novartis Pharmaceuticals), Formestan (purchasable as Lentaron from Novartis) and Exemestane (purchasable as Aromasin from Pharmacia). Aromatase inhibitors may be identified by common enzyme inhibition assay using the aromatase enzyme. As an example such an assay is described in Matsui et al (2005), J Pharm Biomed Anal, 38 (2) :307-12.

According to another aspect, the anti-estrogen treatment comprises administration of an agent which downregulates expression of an estrogen receptor, preferably the estrogen receptor alpha. Preferably, the agent which downregulates expression of an estrogen receptor is Fulvestrant or a functional derivative thereof. Fulvestrant may be obtained under the name Faslodex from Astra Zeneca. Fulvestrant is an estrogen receptor antagonist which blocks estrogen binding to an estrogen receptor, preferably to the estrogen receptor alpha. Additionally, it induces downregulation of the receptor (Morris, C. and Wakeling, A., Endocr Relat Cancer (2002), 9(4): 267-76; Gradishar, W. J., Oncologist. (2004), 9(4): 378-84). Other compounds to trigger downregulation of the estrogen receptor may include small interfering RNAs (siRNAs), targeting specific (e.g. ER) mRNA (see Grunweller et al. (2005), Current Medicinal Chemistry 12(26), 3143-3161),or other modifiers of transcription as reviewed in Melnick et al. (2005), JCO 23(17), 3957-3970).

The anti-estrogen treatment may be performed as a monotherapy or in a combination therapy together with chemotherapy and/or radiation. Preferably, the anti-estrogen treatments are to be performed as a monotherapy. According to one aspect of the invention, an anti-estrogen therapy is performed as an adjuvant therapy in patients afflicted with endometrium or ovary cancer in order to prevent metastases. Anti-estrogen therapy may also be useful for prophylactic treatment of patients at high risk of developing endometrium or ovary cancer in order to prevent cancer development. Anti-estrogen treatment is often used for palliative treatment in women with advanced and recurrent endometrial or ovarian cancer, to extend the symptom-free time, given its excellent tolerability in comparison with chemotherapy.

Normally, tumors of the endometrium or ovary, such as carcinomas, are removed by surgical means in a first therapeutic step which is followed most cases by an adjuvant therapy. At present, several surgical approaches have been established, for example complete or partial hysterectomy or ovariotomy with or without subsequent radiation therapy or chemotherapy. Where surgical removal of the tumor is not possible, for example in cases where invasion of the healthy tissue is in a too advanced state, chemotherapy is often employed to reduce the tumor size before hysterectomy or ovariotomy.

The principal purpose of adjuvant therapy is to eradicate cancer cells that may have spread from the primary tumor and remain after surgical removal of said tumor. Thus, treatment is normally performed systemic, for example, by oral uptake or injection into the bloodstream which allows the agent to circulate through the body. Whether a patient has to be treated with an adjuvant therapy is dependent on the individual risk of the patient to develop metastases at a later stage of the disease and several other factors, such as tumor size, histological type of the tumor as well as the grade of aggressiveness of the disease. On the basis of these factors, patients are attributed to a low, mid or high risk to develop metastases. Adjuvant therapy for endometrium or ovary cancer may comprise anti-estrogen therapy or chemotherapy, either alone or in combination.

In the context of an adjuvant therapy, chemotherapy is commonly performed in accordance with the so-called CNF scheme, using the substances cyclophosphomid, metotrexat and 5fluoruracil. Alternatively, chemotherapeutic regimens based on anthracycline-containing agents are also available. Other frequently used chemotherapeutic agents include alkylating agents, e.g. ethylenimines and methylamelamines, such as thiotepa, altretamine, triethylenemelamine, trietylenephosphoramide, triethylenethiophosphaoramide and trimethylolomelamine; alkyl sulfonates such as busulfan and piposulfan; nitrogen mustards such as ifosfamide, chlorambucil, estramustine, chlornaphazine, cholophosphamide, mechlorethamine, mechlorethamine oxide hydrochlonde, novembichin, phenesterine, prednimustine, trofosfamide; nitrosureas such as fotemustine, lomustine, carmustine, chlorozotocin, nimustine, ranimustine; aziridines such as carboquone, benzodopa, meturedopa, uredopa; purine analogs such as 6-mercaptopurine, fludarabine, thioguanine; pyrimidine analogs such as ancitabine, azacitidine, 6-azauridine, carmofur, cytarabine, dideoxyuridine, doxifluridine, enocitabine, floxuridine, 5-FU; and nitrosureas such as carmustine, chlorozotocin, fotemustine, lomustine, nimustine, ranimustine and the like. However, due to the mainly unspecific mode of action of the respective agents, chemotherapy is a severe treatment with considerable side effects for the patient. Thus, it is highly desirable to identify patients which can effectively treated without or with low-dose chemotherapy. In this context, it is of critical relevance to identify patients which exhibit an enhanced clinical response to anti-estrogen therapy, such as administration of Tamoxifen. Thus, in one aspect the present invention provides a suitable means for identifying a subgroup of estrogen receptor-positive patients which show an enhanced response to anti-estrogen treatment and, therefore, are suitable to be subjected to treatment protocols which are based on the administration of antiestrogens, either without the need for concurrent chemotherapy or in combination with low-dose chemotherapy. Therefore, the invention contributes to a better prognosis as well as to an improved overall condition of the patient during treatment. In this manner, effective treatment can be conducted in a way that is much less associated with the unpleasant and health-threatening effects of chemotherapy and at the same time maintain a high level of medical effect.

The invention also provides a suitable method for the long-term surveillance of the responsiveness of a tumor or patient to anti-estrogen treatment, in which the method for identifying a tumor as responsive to anti-estrogen treatment as described herein is performed sequentially, for example, twice within a period of 3, 6, 9, 12 or 18 month in order to monitor if changes to the amplification status have occured during a particular treatment regimen, for example treatment with Tamoxifen or another anti-estrogen drug. The method will be particularly suitable to evaluate whether a given tumor resulting from a proliferative disease of the endometrium or ovary develops resistance against the anti-estrogen treatment.

Tumors cells with low level amplification, normal ESR1 gene copy numbers, or cells with a loss of ESR1 might not respond optimal to anti-estrogen treatment or even escape such treatment. Such cells are likely to have a selection advantage under anti-estrogen treatment and might be a source for the development of hormone refractory (resistant) tumors. Such an effect is known for topoisomerase 2 alpha, the molecular target of anthracycline therapy. Studies carried out in vitro suggested that cell lines without TOP2A aberrations or those with TOP2A deletion are less sensitive to anthracycline therapy than cell lines with TOP2A amplification or overexpression (Järvinen et al. (2000), Am J Pathol 156:839). Only recently, this observation has been confirmed in a clinical study including 391 breast cancer patients (Scandinavian Breast Group Trial 9401 (2006), J Clin Oncol. 24(16):2428). Accordingly, monitoring of changes in the ESR1 amplification status in sequential biopsies from cancer patients could be a potential marker for prediction of response to anti-estrogen treatment.

The methods of the present invention are based on the predictive impact of ESR1 gene amplification. ESR1 amplification can be conveniently detected by means which directly target the ESR1 coding sequence provided by nucleotides 1048135 to 1343855 in SEQ ID NO:1 or at least a part of this coding sequence. Apart from this, ESR1 amplification can also be confirmed by detecting amplification of a sequence portion of SEQ ID NO:1 which is located outside of the ESR1 coding sequence. Generally, the amplification of a specific gene leads to the co-amplification of genomic sequences which are located 5' or 3' of said gene. Hence, the chromosomal fragment which is duplicated during amplification regularly not only comprises the sequence of a single gene, but also additional genomic sequences which can be used as a marker to confirm ESR1 amplification. As described in example 6, a sequence range having a size of approximately 2,7 Mb (provided in SEQ ID NO:1) which contains the ESR1 coding sequence was found to be suitable for confirming amplification of the ESR1 coding sequence. Specifically, it was found that the sequences located outside the ESR1 coding sequence and being comprised by SEQ ID NO:1 are only amplified, if the ESR1 coding sequence is also amplified, i.e. they are co-amplified with the ESR1 coding sequence. This means that in cases where amplification of a sequence portion of SEQ ID NO:1 is detected, for example, amplification of a sequence located at the very 5' or 3' end of the sequence of SEQ ID NO:1, amplification of ESR1 is to be assumed.

A nucleotide sequence portion located outside the ESR1 coding sequence which can be selected for confirming ESR1 amplification may be located in the region extending from position 1 to position 1048135 of the sequence shown in SEQ ID NO:1. For example, the nucleotide sequence portion may be located between nucleotides 100000 to 1048135, 200000 to 1048135, 300000 to 1048135, 400000 to 1048135, 500000 to 1048135, 600000 to 1048135, 700000 to 1048135, 750000 to 1048135, 760000 to 1048135, 770000 to 1048135, 780000 to 1048135, 790000 to 1048135, 800000 to 1048135, 810000 to 1048135, 820000 to 1048135, 830000 to 1048135, 840000 to 1048135, 850000 to 1048135, 860000 to 1048135, 870000 to 1048135, 880000 to 1048135, 890000 to 1048135, 900000 to 1048135, 910000 to 1048135, 920000 to 1048135, 930000 to 1048135, 940000 to 1048135, 950000 to 1048135, 960000 to 1048135, 970000 to 1048135, 980000 to 1048135, 990000 to 1048135, 1000000 to 1048135, and even more preferably between 1010000 to 1048135, 1020000 to 1048135, 1030000 to 1048135, 1040000 to 1048135, 1041000 to 1048135, 1042000 to 1048135, 1043000 to 1048135, 1044000 to 1048135, 1045000 to 1048135, 1046000 to 1048135, 1047000 to 1048135, 1048000 to 1048135 of the sequence shown in SEQ ID NO: 1. Likewise, the nucleotide sequence portion may be located between nucleotides within the region extending from position 1343855 to 2725892 of the sequence shown in SEQ ID NO:1. For example, the nucleotide sequence portion may be located between nucleotides 1343855 to 1344000, 1343855 to 1345000, 1343855 to 1346000, 1343855 to 1347000, 1343855 to 1348000, 1343855 to 1349000, 1343855 to 1350000, 1343855 to 1351000, 1343855 to 1352000, 1343855 to 1353000, 1343855 to 1354000, 1343855 to 1355000, 1343855 to 1356000, 1343855 to 1357000, 1343855 to 1358000, 1343855 to 1359000, 1343855 to 1360000, 1343855 to 1370000, 1343855 to 1380000, 1343855 to 1390000, 1343855 to 1400000, 1343855 to 1410000, 1343855 to 1420000, 1343855 to 1430000, 1343855 to 1440000, 1343855 to 1450000, 1343855 to 1460000, 1343855 to 1470000, 1343855 to 1480000, 1343855 to 1490000, 1343855 to 1500000, 1343855 to 1510000, 1343855 to 1520000, 1343855 to 1530000, 1343855 to 1540000, 1343855 to 1550000, 1343855 to 1560000, 1343855 to 1570000, 1343855 to 1580000, 1343855 to 1590000, 1343855 to 1600000, 1343855 to 1610000, 1343855 to 1620000, 1343855 to 1630000, 1343855 to 1640000, 1343855 to 1650000, 1343855 to 1660000, 1343855 to 1670000, 1343855 to 1680000, 1343855 to 1690000, 1343855 to 1700000, 1343855 to 1800000, 1343855 to 1900000, 1343855 to 2000000, 1343855 to 2100000, 1343855 to 2200000, 1343855 to 2300000, 1343855 to 2400000, 1343855 to 2500000 or 1343855 to 2725892 of the sequence shown in SEQ ID NO: 1.

If amplification of ESR1 is tested by detecting amplification of a sequence portion of SEQ ID NO:1 which is located outside of the ESR1 coding sequence and no amplification of the selected sequence portion can be detected in a sample, this does not necessarily allows the conclusion that no ESR1 amplification is present in the tested cells. In these cases, the amplicon (i.e. the amplified genomic sequence entity consisting of the ESR1 gene and flanking regions) in the chromosome might be smaller in size, so that further detection assays using nucleotide sequences portions of SEQ ID NO:1 which are in closer vicinity to the ESR1 gene or which are directly derived from the ESR1 gene should be performed. The person of skill will have no problems to determine flanking regions on the 5' end and at the 3' end of the ESR1 gene, respectively, which are obligatory co-amplified in each ESR1 amplification event. Thus, a "minimum" amplicon can be readily determined by the person skilled in the art, simply by screening a high number of tumor cell samples for ESR1 amplification, for example by FISH using a probe which directly binds to the ESR1 coding sequence, and subsequently determining the 5' and 3' ends of the identified amplicons. Once in receipt of such minimum amplicon, it is possible not only to positively confirm an ESR1 amplification on the basis of an amplified sequences outside the coding sequence, but also to exclude ESR1 amplification on the basis of such sequences in case the test result should be negative. In a method using the minimum amplicon, essentially all tumor cells which exhibit ESR1 amplification will be identified. Thus, according to a preferred aspect, a nucleotide sequence portion of the nucleotide sequence of SEQ ID NO:1 for use in the detecting step is chosen which is obligatory co-amplified with the ESR 1 coding sequence.

An alternative possibility to establish a method which identifies all ESR1-amplified tumor cells is to select the ESR1 coding sequence for analysis. Thus, according to a preferred embodiment of the invention, the selected nucleotide sequence portion of SEQ ID NO:1 comprises at least a part of the ESR1 coding sequence ranging from nucleotide positions 1048135 to 1343855 of SEQ ID NO:1. According to this particular embodiment, amplification of at least a part of the ESR1 gene is directly tested. The sequence portion can be selected to include a part of the coding sequence of ESR1 and a part of the flanking regions from the 3' or 5' end of the ESR1 coding sequence. The selected nucleotide sequence may also comprise the complete ESR1 coding sequence, as provided by nucleotide positions 1048135 to 1343855 of SEQ ID NO:1, for example, as a probe to be used in FISH assays. According to a further preferred embodiment of the invention, the selected nucleotide sequence portion of SEQ ID NO:1 is located within the ESR1 coding sequence ranging from nucleotide positions 1048135 to 1343855 of SEQ ID NO:1.

All methods of the present invention comprise the step of detecting in a cell sample from a tumor whether a selected nucleotide sequence portion contained in the sequence according to SEQ ID NO:1, for example the ESR1 coding sequence, is amplified in the genome of the tumor cells. The nucleotide sequence portion of SEQ ID NO:1 selected for this purpose can essentially be of any size. Preferably, nucleotide sequence portions having a size sufficiently large to exclude false-positive detection of amplification, for example by unspecific hybridization, are used. Typically, the sequence portion will have a size of more than 20, 30, 40, 50, 100 or 150 nucleotides. The size of the nucleotide sequence portion will depend on the method of detecting amplification of said sequence portion (see below). For example, if FISH assays are used for amplification detection, the sequence portion will preferably have a size of several kilobases, for example 40, 50, 60, 70, 80 100, 120, 140, 160, 180, 200, 300 or 400 kilobases. In comparison, if amplification detection will be performed by PCR, the sequence portion will have the size of a PCR product which is easy to obtained under standard PCR conditions, for example 150, 200, 300, 400 or 500 bp. If amplification detection is performed by Southern Blotting, the nucleotide sequence portion to be selected can be in the range of 30, 40, 50, 60 nucleotides which corresponds to a common DNA probe regularly used in Southern Blotting.

As used herein, "detecting whether a selected nucleotide sequence portion is amplified" means that it is examined whether the selected nucleotide sequence portion of SEQ ID NO:1 occurs in an increased copy number in the genome of a tested tumor cell relative to the genome of a normal cell with a balanced caryotype, preferably a normal diploid somatic cell of the same individual. Diploid organisms, such as mammals, usually have two copies (allels) of a given genomic nucleotide sequence, such as a gene sequence, in their somatic cells. Hence, a balanced caryotype of a somatic mammalian cell regularly comprises two copies of a given nucleotide sequence, for example of the ESR1 gene.

If the nucleotide sequence portion selected from SEQ ID NO:1 is amplified in the cell sample, more copies of said nucleotide sequence portion are present in the genome of a tested cell when compared to a normal cell with a balanced caryotype. Thus, in cases where the selected nucleotide sequence portion is amplified in the genome of a cell, the copy number of said nucleotide sequence portion exceeds 2. For example, the copy number of said nucleotide sequence portion may be 3, 4, 5, 6, 7, 8, 9, 10, 11, 12, 13, 14, 15, 20, 30, 40, 50 or even up to 100 copies per cell. The copy number of said nucleotide sequence portion can also be decreased relative to the balanced caryotype of a somatic mammalian cell, which means that the cells have lost one or both copies of the nucleotide sequence portion from the relevant chromosome, for example from human chromosome 6. In cases where the nucleotide sequence portion has not been amplified, 2 copies of said sequence should be present per cell.

Typically, amplification of a gene, a gene fragment, or a larger portion of a chromosome comprising more than one gene is determined by assessing the number or intensities of signals obtained (depending on the specific detection method) for the gene, fragment or portion of interest relative to the number or intensities of signals of a reference sequence from the same DNA sample. For example, a sequence for which the copy number is known (i.e. from a gene or a non-coding DNA stretch which does not undergo amplification events) may be used as a reference. The nature of the reference sequence will depend on the specific method of determining the amplification event, e.g. PCR, Southern-Blot, FISH and the like (see below). For example, in fluorescence in situ hybridization assays, the sequence of the centromere of human chromosome 6 or 17 might be conveniently used as an intrinsic reference. Alternatively, if PCR approaches are used for assessing the copy number, reference genes may comprise one or more of the genes commonly used as "housekeeping genes" such as genes encoding human albumin glyceraldehyde 3-phosphate dehydrogenase (GAPDH), β-actin, β-2 microglobulin, hydroxymethylbilane synthase, hypoxanthine phosphoribosylm transferase I, ribosomal protein L13a, succinate dehydrogenase complex (subunit A), TATA box binding protein, ubiquitin C, β-Globin (HBB), Phosphoglycerate kinase 1 (PGK1), Ribosomal protein L4 (RPL4), Large ribosomal protein P0 (RPLP0), Eukaryotic elongation factor 1 (EEF1A1), Eukaryotic translation elongation factor 1 (EEF1G), Succinate dehydrogenase complex A (SDHA), Muscleblind-like 2 (MBNL2), 28S Ribosomal RNA (28S), 18S Ribosomal RNA (18S), and the like. In cases, where an internal reference is simultaneously tested with the cells or DNA of the test samples, e.g. a normal somatic cell with a balanced caryotype, it may not be necessary to determine the specific number of copies of the ESR1 gene as long as it is shown that significantly more detection signals (which are correlated to the copy number of the ESR1 gene) are obtained in the test sample relative to the control sample.

The amplification status of the selected nucleotide sequence portion of SEQ ID NO:1 can be detected according to methods well-known in the art. The amplification status is generally determined by analysis of the genomic DNA of a cell sample. The genomic DNA may be isolated and/or purified before determining the amplification status as usually required for PCR-based methods. For purification, commercially available kits, such as the QIAgen Genomic tip system (QIAgen, Hilden, Germany), may be employed. Other methods for purifying genomic DNA from different, types of cells, such as cells from human tissues are discussed in Sambrook, J. et al. (2001); Molecular Cloning: A Laboratory Manual (Third Edition), Cold Spring Harbor Laboratory Press. On the other hand, if cytogenic methods like fluorescence in situ hybridization (FISH) or immunohistochemistry are used, complete cells or tissue portions may be employed without the need to isolate the DNA in an initial step.

Several methods of identifying gene amplification events and/or determining the copy number of a DNA entity (such as a gene) have been described in the art. According to the present invention, detecting whether said nucleotide sequence portion of SEQ ID NO:1 is amplified comprises DNA analysis using a probe which hybridizes to said nucleotide sequence portion. Most preferably, the probe hybridizes to the ESR1 coding sequence ranging from nucleotide positions 1048135 to 1343855 of SEQ ID NO:1 or a part thereof. As used herein, "hybridization" in the context with a primer or probe means that the primer or probe forms a non-covalent interaction with the target polynucleotide, e.g. the ESR1 gene in the genomic DNA of the cell to be tested or a flanking region thereto which is located on the same amplicon like the ESR1 gene. Preferably, the hybridization is a specific hybridization. As used herein, a specific hybridization of a probe or primer means that the probe or primer substantially only hybridizes with the target DNA sequence to which it shows complementarity and substantially not to unrelated sequences. Specific hybridization of a probe or primer occurs when the level of sequence identity between the probe or primer and the target sequence is sufficiently high. Generally, nucleotide sequences which share about 50, 60, 70 or 80%, more preferably 90 or 95, 96, 97, 98, or 99% sequence identity will specifically hybridize.

A specifically hybridized probe or primer remains hybridized to its target sequence under stringent conditions. As used herein, "stringent conditions" are conditions of temperature and salt that lead to an environment in which substantially only allows a primer or probe to remain hybridized to the target sequence in the case of a substantial sequence identity between the probe or primer and the target sequence. Stringent conditions are sequence dependent and are different under environmental parameters. Generally, stringent conditions are selected to be about 5°C to 20°C lower than the thermal melting point for the specific sequence at a defined ionic strength and pH. The thermal melting point is the temperature (under defined ionic strength and pH) at which 50% of the target sequence hybridizes to a perfectly matched (i.e. perfectly complementary) probe. For example, stringent conditions may include hybridization at a temperature ranging from 42° to 65°C. The hybridization solution and washing buffers used may be of high ionic strength, for example 6 times SSC buffer with or without the addition of SDS or other detergents.

Conditions for nucleic acid hybridization and calculation of stringencies can be found in Sambrook, J. et al. (2001); Molecular Cloning: A Laboratory Manual (Third Edition), Cold Spring Harbor Laboratory Press and Haymes, B. D. et al. (1985) in Nucleic Acid Hybridization, A Practical Approach, IRL Press, Washington, D.C. Moreover, computer programs are available for assisting the constructions of optimal probes and primers, for example, InforMax by Vector NTI (distributed by Invitrogen) or Premier Biosoft by Primer Premier.

A nucleic acid probe or primer used for the detection of the amplification status of a nucleotide sequence portion selected from SEQ ID NO:1 may be a perfect complement of the selected sequence portion or a part thereof (e.g. a sequence within the ESR1 coding sequence or a sequence located in a genomic region flanking ESR1) or may be substantially complementary thereto. A "perfect" complementary probe or primer means that every nucleotide of the probe or primer molecule is complementary to the nucleotide at the corresponding position of the target sequence. A probe or primer is "substantially complementary" to a target sequence if one or more nucleotides in the primer or probe are not complementary to the corresponding nucleotide in the target sequence, whereas a sufficient number of complementary nucleotides exist, so that the specific hybridization can occur.

Specific hybridization of a probe to the corresponding nucleotide sequence portion enables detection of the number of copies of said nucleotide sequence portion in a sample, such as a tissue sample. For this purpose, the probe will comprise a detectable label. Numerous different substances are available in the art for labeling a DNA probe, including compounds or composition detectable by spectroscopic, photochemical, biochemical, immunochemical, electrical, optical or chemical means. The labeling is intended to encompass direct labeling of the probe by coupling (i.e., physically linking) a detectable substance to the probe, as well as indirect labeling of the probe by reactivity with another reagent that is directly labeled. Examples of indirect labeling include end-labeling of a DNA probe with biotin such that it can be detected with fluorescently labeled streptavidin. Alternatively, the probes may be labeled with digoxigenin which can be detected with an anti-digoxigenin antibody which in turn can be labeled or recognized by a labeled secondary antibody. Detectable labels for use in the present invention include magnetic beads (e.g., Dynabeads), fluorescent dyes (such as fluorescein, texas red, rhodamine, CY3, CY5, Alexa dyes, green fluorescent protein, and others), radiolabels (e.g., ³H, ¹²⁵I, ³⁵S, ¹⁴C, or ³²P), enzymes (e.g., horse radish peroxidase, alkaline phosphatase and others commonly used in an ELISA), and colorimetric labels such as colloidal gold (e.g., gold particles in the 40-80 nm diameter size range scatter green light with high efficiency) or colored glass or plastic (e.g., polystyrene, polypropylene, latex, etc.) beads. Numerous other systems and compound s for labeling DNA probes are known in the art.

According to the invention, detecting whether the selected nucleotide sequence portion of SEQ ID NO:1, for example a sequence located within the coding sequence of ESR1, is amplified may comprise Southern-Blotting. Southern blotting is a well established method for locating a particular DNA sequence within a complex mixture. DNA, such as genomic DNA, is digested with a restriction enzyme and separated by gel electrophoresis in an agarose gel. Subsequently, the DNA is transferred from the agarose gel onto a membrane (such as a nylon or nitrocellulose membrane) which is incubated with a labeled DNA probe specific for the sequence to be detected. The location of DNA fragments derived from the genomic DNA that hybridizes with the probe can be displayed by detecting the label. If the ESR1 gene is amplified, at least two fragments (of the same or different size) should be detected in Southern blotting. The probes used in Southern blotting are usually indirectly labeled with molecules that can be detected by systems that provide for an enhanced signal intensity (such as digoxigenin detection by an anti-digoxigenin antibody or biotin detection by horseradish peroxidase-conjugated streptavidin and subsequent exposure to a chemiluminescent substrate). Alternatively, Southern blotting probes are often radiolabled which produces a strong signal. As a consequence, probes used in Southern blotting may be of considerably short length when compared to other hybridization approaches (see below). Usually, the probe will have the size of 15-20 bp, more preferably up to 25, 30, 35, 40, 45, 50 or 55 bp.

According to a particularly preferred aspect, detecting whether the selected nucleotide sequence portion of SEQ ID NO:1, for example a sequence located within the coding sequence of ESR1, is amplified comprises fluorescent in-situ hybridization (FISH). Protocols for conducting FISH analysis with labeled probes are available in the art (see, for example, Cherif et al. (1989) Hum Genet. 1989 Mar;81(4):358 or Hyytinen et al. (1994) Cytometry 16(2):93). In such an in-situ hybridization assay, the cells or tissues to be examined are normally fixed to a solid support, such as a glass slide. Cells may be intact in case of touch preparations of surgical samples or truncated in case of tissue sections. Subsequently, the cells are typically denatured with heat or alkali. The cells are then contacted with a hybridization solution at a moderate temperature to permit annealing of labeled probes specific to the nucleic acid sequence encoding the protein. The probes are typically labeled, e.g., with one or more fluorescent reporters. The targets are then washed at a predetermined stringency or at an increasing stringency until an appropriate signal to noise ratio is obtained. When the fluorescence-labeled nucleic acid probes are hybridized to cellular DNA targets, the hybridized probes can be viewed directly using a fluorescence microscope. By using multiple nucleic acid probes with different fluorescence colors, simultaneous multicolored analysis (i.e., for different genes or sequences) can be performed in a single step on a target cell. Fluorochrome-directly labeled nucleic acid probes eliminate the need for multi-layer detection procedures (e.g., antibody-based system) which allows for fast processing and also reduces non- specific background signals. The fluorochrome-directly labeled nucleic acid probes used in FISH assays are usually longer than those used in Southern-blotting. Conventional fluorescence in situ hybridization (FISH) commonly uses cloned genomic probes for hybridization to fixed, denatured chromosomes. These genomic probes are generally large and most often cloned into vectors, such as cosmids, yeast, or bacterial artificial chromosomes that accept genomic inserts having a size of up to several 100 kilobases. According to the invention, the FISH probe may have a size of 1, 5, 10, 20, 30, 40, 50, 60 or up to 100 kb, or even of 200, 300 or 400 kb. FISH probes may be directly labeled (e.g. by fluorescent dyes) or indirectly labeled (e.g. by a hapten, such as digoxigenin or biotin). According to the invention, it is most preferred to use fluorescent labels, so that the result of the hybridization to the genomic DNA of the test sample (e.g. cells of tissue derived from biopsy) can directly be observed. As a intrinsic reference, the sequence of the centromere of human chromosome 6 or 17 might be conveniently used in FISH assays (see examples). Labeling kits for fluorescence labeling may be obtained from different manufacturers, such as the SpectrumOrange- Spectrum-Green-, and SpectrumRed-labeling kit purchasable by Vysis Inc., Downer's Grove, Illinois, USA. FISH assays have found widespread use in the detection of gene amplification events, for example in the context of detection of the *erb-B2* (*HER-2*/*neu)* gene encoding the orphaned receptor tyrosine kinase Erb-B2 (also referred to as HER-2 or neu) which is reported to be a frequently amplified oncogene in breast cancer. See, for example, the publications of Masood et al. (1998), Ann Clin Lab Sci. 28(4):215, Press et al. (2002) J Clin Oncol. 2002 20(14):3095, all of which are included by reference.

A further alternative to detect a potential amplification of the nucleotide sequence portion selected from SEQ ID NO:1, for example a sequence located within the coding sequence of ESR1, involves Comparative Genomic Hybridization (CHG). This cytogenetic method allows the entire genome to be scanned in a single step for copy number aberrations in chromosomal material.

CGH is described in detail, for example in Kallioniemi O., et al. (1992), Science 258: 818-821 (conventional CGH) or Solinas-Toldo S., et al., (1997), Genes Chromosomes Cancer 4: 399-407 (matrix CGH). In CGH, the complete genomic DNA of a cell or cell population to be examined, such as a tumor cell (test DNA), is used as a probe in a hybridization assay, typically against the genomic DNA of normal healthy cells (reference DNA). Test and reference DNA are differently labeled and co-hybridized on a target matrix which comprises of metaphase chromosome spreads from healthy individuals (conventional CGH) or of an array of defined DNA sequences (cloned human genome fragments of several Kilobases or oligonucleotides; array- or matrix-CGH). It may also be possible to perform CGH without simultaneous co-hybridization of a reference DNA, e.g. when using so-called GeneChips (Affymetrix Santa Clara, CA).

Chromosomal regions which have been gained or lost when compared to the balanced caryotype control DNA can be detected by their increased or decreased staining relative to the general staining of the reference genomic DNA. Regions in an increased copy number give rise to a stronger signal compared to the control DNA. One may also, as a negative control, test a reference DNA from a healthy tissue, preferably tissue of the same part of the body (e.g. healthy endometrial or ovarian tissue). The reference DNA may be derived from the donor of the tumor tissue or from another healthy donor. The alterations are classified as DNA gains and losses and reveal a characteristic pattern that includes copy number changes at chromosomal and subchromosomal levels. The use of CGH for analysis of solid tumors has revealed a number of recurrent chromosomes copy number aberrations including amplifications that had not been detected previously. For example, by use of CGH amplifications at chromosome 3q26-27 and 20q13 in various tumors were detected and led to the identification of target genes, such as *PIK3CA* and *ZNF217* which are amplified in ovarian cancer and breast cancer, respectively. As used herein, the term CGH comprises matrix CGH, array CGH and any other method of comparative genome hybridization using isolated labeled DNA and complementary DNA fixed on a solid surface. Most conveniently, CGH methods are conducted by use of array-based hybridization formats. Arrays typically comprise a multiplicity of different probe or target nucleic acids attached to one or more surfaces. Preferably, the surface is a solid surface, such as polyethylene, polypropylene, polystyrene, nitrocellulose, nylon, glass, quartz, silicones, polyformaldehyde, cellulose, or cellulose acetate. In a preferred embodiment, the multiplicity of nucleic acids (or other moieties) is attached to a single contiguous surface or to a multiplicity of surfaces juxtaposed to each other. In an array format a large number of different hybridization reactions can be run simultaneously. Arrays, particularly nucleic acid arrays can be produced according to a wide variety of methods known to the person of skill, for example by spotting using a pipette or by oligonucleotide synthesis technology. Methods for preparing arrays are described, for example, in Xing, W.L. and Cheng, J. (eds.) Biochips. Technology and Applications, Springer, Berlin 2003.

Aside from hybridization-based assays using labeled probes, amplification of the nucleotide sequence portion selected from SEQ ID NO:1, for example a sequence located within the coding sequence of ESR1, can also be detected by PCR-based methods. Thus according to a further preferred aspect, detecting whether the selected nucleotide sequence portion of SEQ ID NO:1 is amplified comprises a PCR, preferably a quantitative PCR (qPCR). Preferably, the PCR uses at least one primer which hybridizes to the ESR1 coding sequence ranging from nucleotide positions 1048135 to 1343855 of SEQ ID NO:1 or a part thereof.

Protocols for qPCR are known to the person skilled in the art and can be found, for example, in Bartlett and Stirling (2003), PCR Protocols (Methods in Molecular Biology), 2nd edition, Humana Press, Totowa, NJ, USA. Quantitative PCR is a method for multiplying nucleic acid molecules which additionally allows for a quantification of the PCR product. Quantification can be achieved by comparison, after termination of the PCR, of the signal obtained from the product with a standard curve previously generated with control samples from an exogenous sequence of known concentration and/or copy number (see, for example, Bustin, S.A. (2004), A-Z of Quantitative PCR (IUL Biotechnology, No. 5) (Iul Biotechnology Series) International University Line, La Jolla, USA).

Alternatively, an internal standardization using an endogenous housekeeping gene or sequence for calibration purposes may be used as well. In this method, two different genomic sequences are simultaneously co-amplified using two sets of primer pairs in one reaction tube. One sequence belongs to a single copy gene that does not undergo DNA amplifications and serves as an intrinsic reference to measure the relative DNA copy number difference of the test sequence (e.g. the nucleotide sequence portion selected from SEQ ID NO:1). Because in a PCR reaction the amount of DNA product is doubled with each cycle, the total DNA yield at the end of the reaction depends on the amount of template DNA that was initially present in the sample. If the test gene is amplified there will be abundant PCR product of the test gene as compared to the reference gene after PCR. The ratio between the amounts of PCR products of the reference gene and the test gene reflects the copy number difference between the two genes in the tissue sample. Such PCR approach has been described, for example, as double differential polymerase chain reaction (ddPCR) by Brandt B., et al. (1995), Gene 159: 29-34.

Another possibility for quantification resides in the use of a competitive PCR which utilizes an exogenously added standard composed of neutral DNA fragments, flanked by a common target sequence with target specific primers. In this PCR, one set of primers (directed to the target sequence or gene) is used to amplify both the target sequence or gene and the neutral DNA fragment. The neutral DNA fragments compete with the target DNA for the same reagents and thus act as an internal standard. The internal standard is designed to generate a PCR product of a different size than the target gene. The quantitative competitive PCR targets two templates competing for the same primers in the same reaction. By knowing the amount of internal standard added to the reaction, one can determine the amount of target DNA present, in this case the nucleotide sequence selected from SEQ ID NO:1. Different methods and devices may be utilized to determine the amount of PCR products, including gel electrophoresis, capillary electrophoresis, or real time PCR systems.

According to a particular preferred embodiment, detecting whether the selected nucleotide sequence portion of SEQ ID NO:1 is amplified comprises real-time PCR. If real time PCR methods are used, the amount of DNA product may be detected online during the PCR using sequence unspecific fluorescence dyes (e.g. SybrGreen) or sequence specific fluorescence labeled probes (Taqman probes, FRET probes, molecular beacons). Real time quantitative PCR methods are well known to the person of skill and have been described in great detail in the prior art. For an overview, see Bartlett and Stirling (2003), PCR Protocols (Methods in Molecular Biology), 2nd edition, Humana Press, Totowa, NJ, USA.

For the PCR-based methods according to the invention, the oligonucleotide primers used for generating the PCR product are derived from the sequence provided in SEQ ID NO:1. According to a preferred embodiment, at least one primer used in the PCR hybridizes to the coding sequence of the ESR1 gene ranging from nucleotide positions 1048135 to 1343855 of SEQ ID NO:1 or a part thereof. Thus, the primers will generate a product which is located within the ESR1 coding sequence or extends from the ESR1 encoding sequence into the flanking regions. According to a further preferred embodiment, both primers hybridize to the coding sequence of the ESR1 gene, which means that the resulting PCR product (i.e. the nucleotide sequence portion selected from SEQ ID NO:1) is completely located within the ESR1 coding sequence.

According to a further preferred embodiment of the invention, the amplification status of the ESR1 gene can be indirectly detected by immunohistochemistry (IHC) using an antibody directed against the estrogen receptor alpha. Immunohistochemical detection of ER expression is performed on tissue sections from proliferative endometrium or ovary disease. A pathologist is required to analyze staining and to distinguish physiological from non-physiological ER expression. Physiological ER expression (normal ESR1 copy number) is characterized by an inhomogeneous staining pattern with different cell nuclei showing different staining intensities. In addition, ER staining is not found in all cell nuclei. In contrast, ESR1 amplified cells exhibit a homogeneous diffuse and uniformly strong staining in all cell nuclei of the proliferative disease.

The invention also relates to an in-vitro method of determining the responsiveness of metastases of a primary tumor which results from endometrial or ovarian cancer to anti-estrogen treatment is provided. The method comprises the steps of
a) selecting a nucleotide sequence portion comprised by the sequence of SEQ ID NO:1;
b) detecting the amplification status of said nucleotide sequence portion in a cell sample from said tumor;
c) classifying said metastases as responsive to anti-estrogen treatment, if said nucleotide sequence portion in the cell sample is amplified.

There is no differences between primary endometrial or ovarian cancers and their metastases in terms of their ESR1 amplification status. All metastases examined in this regard showed ESR1 amplification when derived from a primary tumor showing ESR1 amplification. Similarly, no metastase was observed that exhibited ESR1 amplification when derived from a primary tumor not showing ESR1 amplification. Thus, the ESR1 amplification status of the primary tumor is representative for its metastases. This finding is of importance because adjuvant anti-estrogen treatment targets residual tumors cells and metastases rather than the primary tumor that usually has been surgically removed from the body.

According to a further aspect of the invention, kits are provided, which are suitable for conducting one of the methods explained above. Specifically, the kits include means and reagents for detecting whether a nucleotide sequence portion of SEQ ID NO:1 is amplified in a cell sample (or a blood or bone marrow sample). For example, the kit can comprise one or more probes which hybridize to a nucleotide sequence portion of the nucleotide sequence of SEQ ID NO:1. In particular, the probe hybridizes to the coding sequence of the ESR1 gene ranging from nucleotide positions 1048135 to 1343855 of SEQ ID NO:1 or a part thereof. Moreover, the kit can comprise further reagents for labeling the probes to allow for the detection of the nucleic acid hybridization complexes. Preferably, reagents for visualizing the complex formed between one or more probes and the target sequence are also provided. According to a further preferred embodiment of the invention, a kit for PCR-based detection methods is provided. Such kit comprise oligonucleotide primers for generating a PCR product having a sequence comprised by the sequence of SEQ ID NO:1. Preferably, the kit comprise at least one primer which hybridizes to the ESR1 coding sequence ranging from nucleotide positions 1048135 to 1343855 of SEQ ID NO:1 or a part thereof. It may also comprise one or more polymerase enzymes, buffers, nucleotides and/or dyes suitable for PCR-based reactions, in particular for quantitative PCR or quantitative real-time PCR reactions. Typically, the oligonucleotide primers are about 10 nt, 50 nt, or 100 nt in length, preferably about 15 nt to 40 nt in length. The oligonucleotide primer exhibits a identity/homology to the sequence shown in SEQ ID NO:1 that is sufficient to allow for amplification of the sequence. Typically, the oligonucleotide primer contains at least 6, more usually 8, 10, 15, 20, 30, 40, 45 or 50 contiguous nucleotides of the nucleotide sequence shown in SEQ ID NO:1, and in particular of the sequence ranging from nucleotide positions 1048135 to 1343855. Oligonucleotide primer or probes may be chemically synthesized.

According to a further aspect, the invention relates to the use of an anti-estrogen compound for the preparation of a medicament for the treatment of a patient having a tumor resulting from a proliferative disease of the endometrium or ovary, wherein the tumor cells have an amplified ESR1 gene in their genomic DNA, i.e. the genome of the tumor cells exhibit an amplified ESR1 gene. According to a preferred embodiment, the proliferative disease of the endometrium or ovary is endometrium cancer or ovary cancer, respectively. The invention therefore provides, amongst other, a medicament for treating a subgroup of ER-positive endometrium cancer or ovary cancer patients which show an significantly increased reaction to anti-estrogen treatment, such as Tamoxifen. As used herein, an anti-estrogen compound is any compound that targets to interfere with the naturally occurring interaction between estrogen and an estrogen receptor, preferably the estrogen receptor alpha. Administration of an anti-estrogen compound results in blocking the signal-transducing function of the estrogen receptor, which effects the estrogen-induced reactions, such as cell proliferation. The anti-estrogen compound may act by competitive inhibition of estrogen binding to an estrogen receptor, preferably the estrogen receptor alpha. When administered in an therapeutically effective amount, anti-estrogen compounds bind to an estrogen receptor, preferably the estrogen receptor alpha, thereby blocking estrogen from binding to the receptor. Anti-estrogen compounds comprise estrogen antagonists, estrogen receptor downregulators or aromatase inhibitors as described above.

According to a further preferred embodiment, the anti-estrogen compound is an estrogen antagonist as defined above. The estrogen antagonist to be used for preparing the medicament is preferably selected from the group of consisting of Tamoxifen, Raloxifene, Clomifene, Toremifene, Trilostane or functional derivatives thereof. Most preferably, the estrogen antagonist is Tamoxifen or a functional derivative thereof. Alternatively, the anti-estrogen compound to be used for preparing the medicament is an agent which interferes with estrogen synthesis, preferably an aromatase inhibitor. The aromatase inhibitor can be selected from the group of Anastrozole, Letrozole, Formestan, Exemestane or functional derivatives thereof. The estrogen antagonist to be used for preparing the medicament can also be an agent which downregulates expression of an estrogen receptor, preferably the estrogen receptor alpha, such as Fulvestrant or a functional derivative thereof. The compounds can be used according to dosis regimens well known in the art.

The invention will become more evident by the following examples which are solely meant to illustrate the invention, and not intended to limit the invention.

### EXAMPLES

Contingency table analysis and Chi-square tests were used to study the relationship between histologic tumor type, grade, stage and gene amplification.

### 1. ESR1 amplification in endometrial and ovarian cancer

### 1.1 Tissues

Two tissue microarrays (TMA) were used in this study. Formalin fixed (buffered neutral aqueous four percent solution), paraffin embedded tumor material was utilized. All slides from all tumors were reviewed by two pathologists to define the histological grade and the histological tumor type. The endometrial cancer TMA was constructed from primary tumors of 368 endometroid cancer patients was used for this study. The median patient age was 69 (range 31-92) years. Raw survival data were either obtained from the cancer registry of the University of Basel or collected from the patients attending physicians. The mean follow up time was 20 months (range 1-173). The pathologic stage, grade, tumor diameter, and nodal status were obtained from the primary pathology reports. The ovarian cancer TMA was constructed from 297 primary ovarian cancer of different histological subtypes. The median patient age was 58 (range 24-84) years. Conventional large sections from 15 patients with complex endometrial hyperplasias but no endometrial cancer were selected from the archives of the Institute of Pathology, University Clinical Center Hamburg Eppendorf.

### 1.2 Fluorescence in situ hybridization (FISH)

The TMA sections were treated according to the Paraffin Pretreatment Reagent Kit protocol (Vysis, Downers Grove, IL) before hybridization. FISH was performed with a digoxigenated BAC probe (BAC RP11-450E24, RZPD, Germany) covering part of the ESR1 gene. The sequence of the probe corresponded to the sequence ranging from nucleotide 1.064.232 to nucleotide 1.203.918 of SEQ ID NO:1. As a reference, a Spectrum-Orange labeled chromosome 6 centromeric probe (CEP6) purchased from Vysis was used. Hybridization and posthybridization washes were according to the "LSI procedure" (Vysis). Probe visualization using fluorescent isothiocyanate (FITC)-conjugated sheep anti-digoxigenin (Roche Diagnostics, Rotkreuz, Switzerland) was as described (Wagner, U., et al. (1997), Am J Pathol, 151: 753-759). Slides were counterstained with 125 ng/ml 4',6-diamino-2-phenylindole in an antifade solution.

The number of fluorescence signals was estimated in each tissue spot for the centromere 6 and the ESR1 gene probes. ESR1 alterations were defined based on the ratio of gene copy numbers of ESR1 and centromere 6. Tissues with more at least two-fold more ESR1 than centromere 6 copies (ratio ≥ 2.0) were considered "ESR1 amplified". Tissues with more ESR1 than centromere 6 copies not reaching the criteria for amplification were considered "ESR1 gained" (ratio > 1.0 but < 2.0). All other analyzable tissues (Ratio 1.0) were considered "ESR1 normal".

### 1.3 Immunohistochemistry

Immunohistochemical detection of the estrogen receptor alpha protein was performed using the above described TMAs and antibody NCL-L-ER-6F11 as a primary antibody (Novocastra, Newcastle, UK). The TMA slides were deparaffinized and incubated in a pressure cooker at 120°C for 12 min in pH 6 citrate buffer (Retrievit 6 #BS-1006-00, BioGenex, San Ramon, CA). After blocking of endogeneous peroxidase, pre-diluted (1:1000) primary antibody was applied and the slides were incubated overnight at 4°C. The Vectastain ABC Elite system was used for detection of antibody binding. IHC scoring was performed according to the Allred score (Harvey, J. M., et al. (1999), J Clin Oncol, 17: 1474-1481). In brief, intensity of the estrogen receptor staining was recorded in a 4-step scale (0-3) and the fraction of ER positive tumor cells in a 5-step (1-5) scale. Combination of both parameters results in an 8-step score, where all samples with score >2 are regarded as ER positive.

### 1.4 Results

ESR1 FISH analysis was successful in 176/368 (48%) arrayed tissue samples of endometrial cancer. Missing results were either due to missing tissue samples on the TMA (n=56, 15%) or lack of FISH signals (n=136, 37%). ESR1 amplification (ratio ESR1:cen6 ≥ 2.0) was found in 40 (22.7%) tumors and in 3 of 15 (20%) complex hyperplasias. Another 10 (5.7%) cancers showed ESR1 gains. Amplifications were usually low level with 4-9 FISH signals. Only 8 amplified tumors had ≥ 10 (max. 15) ESR1 gene copies. As shown in table 1, ESR1 amplification was unrelated to histopathological parameters including histological subtype, tumor stage (p=0.6167), and grade (p=0.4763). ESR1 copy number changes were also unrelated to patient prognosis.

Further, ESR1 copy numbers were analyzable in 162 ovarian cancers. Analysis failed in the remaining cases because of lack of interpretable FISH signals (n=77), or lack of tumor cells in the tissue sample (n=58). ESR1 amplification was found in 12 of 162 (7.4%) interpretable cases. One additional ovarian tumor showed a gain of ESR1 copy numbers. ESR1 amplifications were particular frequent in the subtype of endometroid ovarian cancers as compared to papillary cancers (15.2%, p=0.09). All amplifications were low level with 4-6 (median 4.5) ESR1 gene copies. The results are summarized in table 4.

Immunohistochemistry was applied to analyze ER protein expression levels. Analysis was successful in 299/368 (81%) arrayed samples. No result was obtained because of lack of tumor cells in the tissue spots (n=20, 5%) or missing tissue spots (n=49, 14%). More than 95% of tumors showed at least weak ER expression. Strongest staining (score 7-8 according to Allred) was found in 114/299 (38%) of samples, and was linked to low grade tumors (p=0.0266). ER expression was unrelated to patient prognosis. All IHC results are summarized in table 2.

To investigate the impact of ESR1 amplification on ER protein levels ESR1 gene copy numbers were compared to immunohistochemical ER protein expression levels. Data on both FISH and IHC were available from 176 tumors. ER expression (score 3-8) was significantly linked to ESR1 amplified tumors (p=0.0026). Moderate to strong ER expression (score 5-8) was found in 95% of ESR1 amplified tumors, but in only 68% of tumors without ESR1 copy number changes. The association between ESR1 copy numbers and ER expression levels is shown in table 3.

## Claims

1. In-vitro method of identifying a tumor resulting from a proliferative disease of the endometrium or ovary as responsive to anti-estrogen treatment, comprising
a) selecting a nucleotide sequence portion of the nucleotide sequence of SEQ ID NO:1;
b) detecting in a cell sample from said tumor whether said nucleotide sequence portion is amplified in the genome of said tumor cells;
c) classifying said tumor as responsive to anti-estrogen treatment, if the nucleotide sequence portion is amplified in the genome of said tumor cells.

2. In-vitro method of identifying a candidate patient with a tumor resulting from a proliferative disease of the endometrium or ovary as suitable for anti-estrogen treatment, comprising
a) selecting a nucleotide sequence portion of the nucleotide sequence of SEQ ID NO:1;
b) detecting in a cell sample from said tumor whether said nucleotide sequence portion is amplified in the genome of said tumor cells;
c) classifying said patient as one that is suitable for anti-estrogen treatment, if the nucleotide sequence portion is amplified in the genome of said tumor cells.

3. Method according to claims 1 to 2, wherein the anti-estrogen treatment comprises administration of an estrogen antagonist.

4. Method according to claim 3, wherein the estrogen antagonist is selected from the group consisting of Tamoxifen, Raloxifene, Clomifene, Toremifene, Trilostane or functional derivatives thereof.

5. Method according to claim 4, wherein the estrogen antagonist is Tamoxifen or a functional derivative thereof.

6. Method according to claims 1 to 2, wherein the anti-estrogen treatment comprises administration of an agent which interferes with estrogen synthesis.

7. Method according to claim 6, wherein the agent which interferes with estrogen synthesis is a aromatase inhibitor.

8. Method according to claim 7, wherein the aromatase inhibitor is selected from the group of Anastrozole, Letrozole, Formestan, Exemestane or functional derivatives thereof.

9. Method according to claims 1 to 2, wherein the anti-estrogen treatment comprises administration of an agent which downregulates expression of an estrogen receptor.

10. Method according to claim 9, wherein the agent which downregulates expression of an estrogen receptor is Fulvestrant or a functional derivative thereof.

11. Method according to any of the preceding claims, wherein the anti-estrogen treatment is to be performed as a monotherapy.

12. Method according to claims 1 to 11, wherein the proliferative disease of the endometrium is endometrial cancer.

13. Method according to claims 1 to 11, wherein the proliferative disease of the ovary is ovarian cancer.

14. In-vitro method of identifying an individual with a tumor resulting from a non-cancerous proliferative disease of the endometrium or ovary who is at risk of developing endometrial or ovarian cancer, respectively, comprising
a) selecting a nucleotide sequence portion of the nucleotide sequence of SEQ ID NO:1;
b) detecting in a cell sample from said tumor whether said nucleotide sequence portion is amplified in the genome of said tumor cells;
c) classifying said individual as one that is at risk of developing endometrial or ovarian cancer, respectively, if the nucleotide sequence portion is amplified in the genome of said tumor cells.

15. Method according to claims 1 to 14, wherein said nucleotide sequence portion comprises at least a part of the ESR1 coding sequence ranging from nucleotide positions 1048135 to 1343855 of SEQ ID NO:1.

16. Method according to claim 15, wherein said nucleotide sequence portion is located within the ESR1 coding sequence ranging from nucleotide positions 1048135 to 1343855 of SEQ ID NO:1.

17. Method according to claims 1 to 16, wherein detection whether said nucleotide sequence portion is amplified comprises DNA analysis using a probe which hybridizes to said nucleotide sequence portion.

18. Method according to claim 17, wherein the probe hybridizes to the ESR1 coding sequence ranging from nucleotide positions 1048135 to 1343855 of SEQ ID NO:1 or a part thereof.

19. Method according to claim 17, wherein the probe comprises a detectable label.

20. Method according to claims 1 to 19, wherein detecting whether said nucleotide sequence portion is amplified comprises Southern-Blotting.

21. Method according to claims 1 to 19, wherein detecting whether said nucleotide sequence portion is amplified comprises fluorescent in-situ hybridization (FISH).

22. Method according to claims 1 to 19, wherein detecting whether said nucleotide sequence portion is amplified comprises a PCR.

23. Method according to claim 22, wherein the PCR uses at least one primer which hybridizes to the ESR1 coding sequence ranging from nucleotide positions 1048135 to 1343855 of SEQ ID NO:1 or a part thereof.

24. Method according to claims 22 to 23, wherein detecting whether said nucleotide sequence portion is amplified comprises quantitative PCR.

25. Method according to claim 24, wherein detecting whether said nucleotide sequence portion is amplified comprises quantitative real-time PCR.

26. Kit for performing a method of one of the claims 1 to 25, comprising means for detecting whether a nucleotide sequence portion of SEQ ID NO:1 is amplified.

27. Kit according to claim 26, comprising a probe which hybridizes to a nucleotide sequence portion of the nucleotide sequence of SEQ ID NO:1.

28. Kit according to claim 27, wherein the probe hybridizes to the coding sequence of the ESR1 gene ranging from nucleotide positions 1048135 to 1343855 of SEQ ID NO:1 or a part thereof.

29. Kit according to claim 28, further comprising reagents for labeling the probes to allow for the detection of the nucleic acid hybridization complexes.

30. Kit according to claim 26, comprising oligonucleotide primers for generating a PCR product having a sequence comprised by the sequence of SEQ ID NO:1.

31. Kit according to claim 30, comprising at least one primer which hybridizes to the ESR1 coding sequence ranging from nucleotide positions 1048135 to 1343855 of SEQ ID NO:1 or a part thereof.

32. Kit according to claim 31, further comprising one or more polymerase enzymes, buffers, nucleotides and/or dyes suitable for PCR-based reactions.

33. Use of an anti-estrogen compound for the preparation of a medicament for the treatment of a patient having a tumor resulting from a proliferative disease of the endometrium or ovary, wherein the tumor cells have an amplified ESR1 gene in their genomic DNA.

34. Use according to claim 33, wherein the proliferative disease of the endometrium or ovary is endometrium cancer or ovary cancer.

35. Use according to claim 33 or 34, wherein the anti-estrogen compound is selected from the group consisting of Tamoxifen, Raloxifene, Clomifene, Toremifene, Trilostane or functional derivatives thereof.

36. Use according to claim 33 or 34, wherein the anti-estrogen compound is selected from the group consisting of Anastrozole, Letrozole, Formestan, Exemestane or functional derivatives thereof or Fulvestrant or a functional derivative thereof.
